# EUROPEAN PATENT APPLICATION

(11) **EP 4 245 848 A1**
(43) Date of publication of application: **20.09.2023**
(21) Application number: 22305300.0
(22) Date of filing: 15.03.2022
(51) Int. Cl.: C12N 9/02, A23L 33/21, C08B 37/00, C12H 1/02, C12C 5/02, C12C 7/28, C12P 19/04, C12R 1/645

(54) **CLARIFICATION WITH LACCASE AND FUNGAL B-GLUCANS**

(71) Applicant: Green Spot Technologies, 31520 Ramonville-Saint-Agne (FR)
(72) Inventor: MOLINA, Manon, 31400 Toulouse (FR); VILLAS-BOAS, Silas, 1145 Luxembourg (LU); GRANUCCI, Ninna, 31400 Toulouse (FR)
(74) Representative: Santarelli

(57) **Abstract**

The present invention relates to a process for clarifying a liquid obtained from a plant material, wherein said process involves the use of laccase and fungal β-glucans.

## Description

The present invention relates to a process for clarifying a liquid obtained from a plant material, wherein said process involves the use of laccase and β-glucans.

Clarification is an industrial process aiming at separating the solid from the liquid in order to offer to the consumer a clear and stable product. Indeed, liquid obtained from a plant material, such as fruit juices and plant-based milks, are naturally cloudy (turbid) and viscous due to the presence of pectin and other polysaccharides. They also contain polyphenols that are responsible for the maderization process leading to turbidity increase, aroma and flavor alteration and formation of haze or sediments. Such processes can occur during storage.

Usually, clarification means decreasing fibers, such as pectin, and decreasing polyphenols, since they are considered to contribute to the cloudiness of juice.

However, the inventors surprisingly found that it was possible to clarify a beverage by contacting it with dietary fibers, *i.e.* fungal β-glucans, and an enzyme, *i.e.* laccase. Indeed, as demonstrated in Example 1 below, fungal β-glucans do not add to pre-existing turbidity, and synergize with laccase to clarify a liquid such as a fruit juice.

The invention offers a new possibility to perform the clarification process that involves the addition of a compound with interesting nutritious effect. Indeed, fungal β-glucans are dietary fibers, as the β configuration cannot be digested by human endogenous enzymes of the gastrointestinal tract. Preferentially, the fungal β-glucans according to the invention are linear (1,3)-β-D-glucose polysaccharides with (1,6)-linked-β-glucosyl or β-(1,6)-oligoglucosyl side chains.

Additionally, they were found to have a great potential of therapeutic properties including decreasing cardiovascular diseases risk, reviewed recently (Wouk et al., Int. J. Biol. Macromol. 177, April 2021, 176-203). The invention has thus the advantage to improve the quality of the end-product combined with the liquid clarification. The addition of dietary fibers brings the liquid obtained from a plant material back to the whole plant in terms of nutritional profile. Fungal β-glucans are also stable at room temperature contrary to enzymes for which storage conditions can be more constraining. Finally, fungal β-glucans enhance laccase potential for clarification.

In addition, Example 2 below shows that the fermented powders produced similarly to the process described in US 2018/0146688 A1, which contain laccase and fungal β-glucans, have a clarification effect too. The resulting juice is of higher nutritional value (vitamins, minerals, fibers addition) and stable. Moreover, the use of such fermented powders that result from the fermentation of by-products from the agro-industry, such as juice industry, (*e*.*g*. apple, grape or orange by-products) paves the way to a circular economy where the whole fruit will contribute to juice processing: its edible part for the juice itself, and its by-product for clarification using fermented powders.

It is an object of the invention to provide a process for clarifying a liquid obtained from a plant material, comprising a step of incubating the liquid with laccase and fungal β-glucans.

By "clarifying a liquid", it is intended to make a liquid clearer, in particular by decreasing the amount of suspended solids in the liquid to be clarified. Preferably, the clarification step efficiently decreases the turbidity and/or the total phenolic content of the liquid to be clarified. More preferably, the clarification step efficiently decreases the total phenolic content. The turbidity of a beverage can be determined by measuring the absorbance of said liquid at 650 nm. Preferably, the turbidity is determined as indicated in Example 2.4 below. The total phenolic content can be determined by following the protocol of Magalhaes et al., Talanta 83 (2010) 441-447; in this case, the total phenolic content values are expressed as equivalent of gallic acid. Preferably, the total phenolic content is determined as indicated in Example 1.3 below.

By "liquid", it is intended a substance, which is liquid in normal temperature and pressure (NTP) conditions.

By "liquid obtained from a plant material", it is meant any liquid obtained from a starting material being a plant. The liquid can be any liquid, such as raw juices, beverages (such as fruit juice, plant-based milk, fermented juices such as wines and beers, etc.), broths and soups.

By "raw juice", it is intended a juice that was never altered from its original form, as in cooked, heated or steamed. Said raw juice can be provided as a concentrate to be diluted or further processed to obtain a beverage.

Preferably, the liquid to be clarified is a juice (such as a raw juice or a beverage), such as a fruit juice, more preferably an apple juice.

By "wine", it is intended a beverage obtained from alcoholic fermentation of fruit raw juices or musts.

By "beer", it is intended a beverage obtained from the brewing process, during which fermentation of the starch sugars in the wort produces ethanol and carbonation in the resulting beer.

By "wort", it is meant a liquid obtained from malt of cereal grains. Preferentially, wort is obtained from malted barley, wheat, maize, rice, oat, sorghum, millet or buckwheat, preferably from malted barley.

By "broth", it is intended a flavorful liquid preferably made by cooking (e.g. simmering) a plant material in water; the majority of the cooked plant material is then removed from the water, which leads to a liquid comprising solid residues of plant material. The broth can be drunk alone or used to prepare other dishes (e.g. sauces, risotto, dumplings, casseroles, stuffing, cooked grains and legumes, gravies, sautéed or stir-fried dishes, etc).

Contrary to a broth, the soup is obtained from the cooked plant material that is ground with water.

By "plant material", it is meant advantageously fruits, vegetables, nuts, seeds and/or cereals, preferably fruits and/or vegetables. Such a plant material can be as a whole, cut into pieces, in the form of a pulp, dehulled or even sprouted. Preferably, the fruits are selected from the group consisting of apple, pears, citrus (such as oranges, grapefruits, mandarins and limes), stone fruits (such as nectarines, apricots, peaches and plums), tropical and exotic fruits (such as bananas, kiwifruit and mangoes), berries (such as strawberries, raspberries, blueberries, kiwifruit and passionfruit), melons. More preferably, the fruit is selected from the group consisting of apple, orange and grape, even more preferably, the fruit is apple. Preferably, the vegetables are selected from the group consisting of leafy greens (such as lettuce, spinach and silverbeet), cruciferous (such as cabbage, cauliflower, Brussels sprouts and broccoli), marrows (such as pumpkin, cucumber and zucchini), roots (such as potato, carrots, turnips, sweet potato and yam), edible plant stems (such as celery and asparagus), tomatoes. Preferably, the nuts are selected from the group consisting of almonds, brazil nuts, cashew nuts, hazelnuts, macadamias, pecans, chestnuts, pine nuts, chufa, pistachios, walnuts, peanuts and coconuts. Preferably, the seeds are selected from the group consisting of chia seeds, flax seeds, hemp seeds, sesame seeds, quinoa, sunflower seeds. Preferably, the cereals are selected from the group consisting of barley, fonio, maize, millet, soy, oat, rice, rye, sorghum, teff, triticale, spelt and wheat. More preferably, soy, oat, rice and spelt, even more preferably, the cereal is soy, oat or rice.

The plant material of a raw juice is preferably fruits and/or vegetables, said fruits and/or vegetables being as described above for the clarification process. In particular, the fruits and/or vegetables are more preferably selected from the group consisting of apple, orange and grape, and can be provided whole or into pieces.

Thus, the liquid obtained from a plant material can be obtained from the plant material with or without addition of water. The liquid can be obtained, for example, by extracting a raw juice from the plant material, *i.e.* by pressing, crushing, squashing and/or centrifuging the plant material. The skilled person knows the conditions to extract raw juice by pressing, crushing, squashing and/or centrifuging. Said raw juice can be further processed into a juice and/or macerated to be fermented. For example, fruit raw juices and grape musts can be fermented to obtain ciders and wine, such as apple, grape or elderberry wine, preferably, grape wine.

The liquid can also be obtained by pressing, grounding, cutting, mixing and/or cooking the plant material with water, in order to obtain a liquid such as plant-based milks, soups or broths. The plant material can also be macerated with additional water, in order to obtain fermented beverages. For example, beer worts can be fermented to obtain beers.

Preferably, the step of obtaining a liquid from a plant material is a step of pressing, crushing, squashing and/or centrifuging fruits and/or vegetables to obtain a ready-to-consume fruit and/or vegetable juice, or a step of pressing crushing, squashing and/or centrifuging, and fermenting fruits to obtain wine (such as grape to obtain grape wine).

More preferably, the step of obtaining a liquid from said plant material is a step of pressing or centrifuging fruits and/or vegetables to obtain a fruit and/or vegetable raw juice.

Advantageously, the liquid to be clarified is a juice (such as a fruit and/or a vegetable raw juice), plant-based milk, soup, broth, beer or wine (such as apple, grape or elderberry wine, preferably, grape wine).

By "laccase", it is intended a multicopper oxidase, which property is to oxidize a variety of phenolic substrates, performing one-electron oxidations, leading to crosslinking. Laccase is designated under the EC number 1.10.3.2. Its activity is preferably measured by monitoring the 2,2'-azinobis(3-ethylbenzthiazoline-6-sulfonate) oxidation at 420 nm, as described in Example 1.2 below.

By "β-Glucans", it is intended a group of β-D-glucose polysaccharides, arranged in six-sided D-glucose rings connected linearly by β-(1,3), β-(1,4) and/or β-(1,6) bonds, and including a 1-3 glycosidic link in their backbone. β-glucans are preferably quantified using aniline blue method, preferably by following the protocol described in Example 1.2 below. The β-glucans implemented in the process according to the invention (for contacting the liquid to be clarified and/or its starting material) are fungal β-glucans. Preferably, the process for clarifying a liquid according to the invention comprises a step of contacting laccase and fungal β-glucans with the plant material, before the plant material is processed into a liquid.

By "contacting" laccase and fungal β-glucans with the plant material, it is preferably intended that said plant material is put into the same container as laccase and fungal β-glucans, in any order. For example, laccase and fungal β-glucans can be added to said plant material, or said plant material can be added to laccase and fungal β-glucans. The clarification step starts once the liquid to be clarified is obtained and thus when this liquid is in contact with laccase and fungal β-glucans. Preferably, the clarification step ends once laccase is inactivated. It should be noted that, by deactivation of laccase, it is intended that laccase activity cannot be detected anymore in the clarified liquid.

Advantageously, the liquid to be clarified is contacted with 0.0001U to 3U of laccase per mL of said liquid, preferably 0.0005 to 1 U/mL, more preferably 0.0015 to 0.30U/mL, even more preferably 0.003 to 0.06U/mL. It is understood that, throughout the text of the present application, "U" (i.e. enzyme unit) represents the amount of the enzyme (i.e. laccase) that catalyzes the conversion of one micromole of substrate per minute. Preferably, 1U represents the amount of laccase oxidizing 1µmol of 2,2'-azinobis(3-ethylbenzthiazoline-6-sulfonate) (ABTS) per minute at pH 5 and about 21°C, for example as described in Example 1.2 below.

It will be noted that in the context of the present application, and unless otherwise stipulated, the ranges of values indicated are understood to be inclusive. By "about a value of X", it is intended more or less 10% of the value of X.

According to an embodiment of the invention, the process for clarifying a liquid comprises or consists in the following steps:
- providing a plant material,
- contacting laccase and fungal β-glucans with the plant material, (i.e. before a liquid is obtained from said plant material),
- obtaining the liquid from said plant material, which thus comprises laccase and fungal β-glucans,
- incubating the liquid comprising laccase and fungal β-glucans to clarify the liquid,
- optionally, filtrating the liquid, in order to retain the remaining solids,
- optionally, inactivating the enzyme,
- optionally, cooling the liquid.

Alternatively or in addition to the step of contacting laccase and fungal β-glucans with the plant material before the plant material is processed, the process for clarifying a liquid according to the invention can comprise a step of contacting laccase and fungal β-glucans with the liquid obtained from the plant material.

The step of contacting with laccase and fungal β-glucans can also be carried out directly on the liquid obtained from the plant material. In particular, when the liquid to be clarified is a raw juice, the step of contacting with laccase and fungal β-glucans is preferably carried out after the extraction of the raw juice (e.g. by crushing, squashing, pressing and/or centrifuging).

According to another embodiment, the process for clarifying a liquid comprises or consists in the following steps:
- providing a plant material,
- optionally, contacting laccase and fungal β-glucans with the plant material, (i.e. before a liquid is obtained from said plant material),
- obtaining the liquid from said plant material,
- contacting laccase and fungal β-glucans with the liquid obtained from said plant material,
- incubating the liquid comprising laccase and fungal β-glucans to clarify the liquid,
- optionally, filtrating the liquid, in order to retain the remaining solids,
- optionally, inactivating the enzyme,
- optionally, cooling the liquid.

According to a preferred embodiment, the process for clarifying a liquid comprises or consists in the following steps:
- providing a plant material,
- obtaining the liquid from said plant material,
- contacting laccase and fungal β-glucans with the liquid obtained from the plant material,
- incubating the liquid comprising laccase and fungal β-glucans to clarify the liquid,
- optionally, filtrating the liquid, in order to retain the remaining solids,
- optionally, inactivating the enzyme,
- optionally, cooling the liquid.

Advantageously, in the process for clarifying a liquid according to the invention, the liquid is a beverage. Preferably, the beverage to be clarified is a juice, beer or wine.

In the context of the invention, it is intended by "beverage" any liquid for drinking other than water. Such beverage can be obtained by modifying a liquid obtained from a plant material as described above, by adding, for example, acidity regulators, antifoaming agents, antioxidants, bulking agents, colors, emulsifiers, flavor enhancers, preservatives, stabilizers, sweeteners, thickeners. Preferably, a beverage according to the present invention is a juice, a plant-based milk, a soup, a broth, wine, cider or beer. More preferably, a beverage according to the present invention is a juice.

According to a preferred embodiment, the process for clarifying a liquid, the liquid being a beverage, comprises the following steps:
- providing a plant material,
- obtaining the liquid from said plant material,
- preparing a beverage from the liquid from said plant material,
- contacting laccase and fungal β-glucans with the beverage,
- incubating the beverage comprising laccase and fungal β-glucans to clarify the beverage,
- optionally, filtrating the beverage, in order to retain the remaining solids,
- optionally, inactivating the enzyme.

Advantageously, when the beverage to be clarified is a beer or a wine, the step of contacting with laccase and fungal β-glucans is preferably carried out after fermentation respectively of wort, must or fruits.

Preferably, the beverage to be clarified was not submitted to any other clarification step before being contacted with laccase and fungal β-glucans.

Advantageously, the liquid to be clarified has a pH comprised between 1 and 7, preferably, between 2 and 6, more preferably, between 3 and 5, even more preferably, of about 4.

Advantageously, the liquid to be clarified is contacted with 0.0002 g to 0.10 g of fungal β-glucans per liter of said liquid, preferably, 0.001 to 0.050 g/L, more preferably, 0.002 to 0.025 g/L, even more preferably, 0.002 to 0.009 g/L.

Preferably, in the process for clarifying a liquid according to the invention, the fungal β-glucan / laccase ratio is comprised between 0.001 g/kU and 5.0 g/kU.

The fungal β-glucan / laccase ratio is expressed in grams of fungal β-glucans per kilo laccase units (1 kU of laccase representing 1000 U of laccase).

Advantageously, the liquid to be clarified is contacted with 0.0001 U to 3 U of laccase per mL of said liquid and with 0.0002 g to 0.10 g of fungal β-glucans per L of said liquid, preferably 0.0005 to 1.0 U of laccase per mL and 0.001 to 0.050 g of fungal β-glucans per L, more preferably 0.0015 to 0.30 U of laccase per mL and 0.002 to 0.025 g of fungal β-glucans per L, even more preferably 0.003 to 0.06 U of laccase per mL and 0.002 to 0.009 g of fungal β-glucans per L.

Laccase and fungal β-glucans can be provided separately or as a combined preparation.

In a preferred embodiment, in the process for clarifying a liquid according to the invention, the laccase and fungal β-glucans can be provided under the form of a fermented powder.

By "fermented powder", it is intended a powder that is agglomerated or not, obtained by fungal fermentation (fungal fermented powder). Said powder can be a powder, extrudates, texturized, in a form of pellets, etc. Said powder can be obtained by fermentation, preferably solid-state fermentation, of a plant material with edible fungi, preferably with edible higher fungi, and more preferably with white rot fungi and/or brown rot fungi, more preferably with a white rot fungi. As indicated above, the fermented powder contain fungal β-glucans, which are linear (1,3)-β-D-glucose polysaccharides with (1,6)-linked-β-glucosyl or β-(1,6)-oligoglucosyl side chains.

Preferably, the nature of the plant material used in the preparation of the fermented powder is as mentioned below, except that it can be preferably a by-product such as pomaces, skins, peels etc.

Advantageously, edible fungi are selected from the group consisting of *Pleurotus* (such as *P*. *ostreatus, P. eryngi, P. pulmonarius, P. djamor, P. australis, P. purpureo-olivaceus, P. citrinopileatus, P. sajor-caju, P. florida, P. flabellatus, P. ferulae, P. cystidiosus*), *Lentinula* (such as *L. edodes, L. boryana, L. novae-zelandiae, L. tigrinus*), *Ganoderma* (such as *G. lucidum, G. applanatum, G. tsugae*), *Volvariella* (such as *V. volvacea, V. esculenta, V. bakeri, V. dysplasia*), *Auricularia* (such as *A. auricola, A. cornea, A. subglabra*), *Armillaria* (such as *A. mellea, A. ostoyae, A. gemina, A. calvescens, A. Sinapin, A. gallica*), *Flammulina* (such as *F. velutipes, F.* fennae), *Pholiota* (such as *P*. *squarrosa, P. nemako*), *Tremella* (such as *T. mesenterica*, *T. fuciformis*), *Hericium* (such as *H. erinaceus, H. coralloides*), *Agaricus* (such as *A. campestris, A. bisporus, A. bitorquis*), *Laetiporus* (such as *L. sulphurous*), *Sparassis* (such as *S*. *crispa*, *S. spathulata*), *Morchella* (such as *M. esculenta, M. hortensis, M. conica, M. vulgaris, M. rotunda, M. costada)*, *Hypsizygus* (such as *H. tessulatus, H. ulmarius*), *Grifola* (such as *G*. *frondosa, G. umbellata)*, *Boletus* (such as *B*. *edulis, B. aereus, B. estivalis, B. pinicola), Stropharia* (such as *S*. *rugosoannulata*), *Cantharellus* (such as *C*. *cibarius, C. edulis)*, *Russula* (such as *R. cyanoxantha, R. virescens, R. vesca), Cyclocybe* (such as *C. aegerita*), *Rhizopus* (such as *R. oligosporus, R. oryzae, R. stolonifer)*, *Aspergillus* (such as *A. acidus, A. niger, A. oryzae, A. sojae)*, *Monascus* (such as *M. albidus, M. anka, M. araneosus, M. major, M. purpureus, M. rubiginosus, M. vini), Penicillium* (such as *P*. *camemberti, P. glaucum, P. nalgiovense, P. roqueforti), Fusarium* (such as *F*. *venenatum)* and *Norospora* (such as *N. intermedia).*

Preferably, edible higher fungi are white rot fungi and/or brown rot fungi selected from the group consisting of *Pleurotus* (such as *P*. *ostreatus, P. eryngi, P. pulmonarius, P. djamor, P. australis, P. purpureo-olivaceus, P. citrinopileatus, P. sajor-caju, P. florida, P. flabellatus, P. ferulae, P. cystidiosus*), *Lentinula* (such as *L. edodes, L. boryana, L. novae-zelandiae, L. tigrinus*), *Ganoderma* (such as *G. lucidum, G. applanatum, G. tsugae*), *Volvariella* (such as *V. volvacea, V. esculenta, V. bakeri, V. dysplasia*), *Auricularia* (such as *A. auricola, A. cornea, A. subglabra*), *Armillaria* (such as *A. mellea, A. ostoyae, A. gemina, A. calvescens, A. Sinapin, A. gallica*), *Flammulina* (such as *F*. *velutipes, F.* fennae), *Pholiota* (such as *P*. *squarrosa, P. nemako*), *Tremella* (such as *T. mesenterica, T. fuciformis*), *Hericium* (such as *H. erinaceus, H. coralloides*), *Agaricus* (such as *A. campestris, A. bisporus, A. bitorquis), Laetiporus* (such as *L. sulphurous*) and *Sparassis* (such as *S*. *crispa*, *S. spathulata*), more preferably, *P. pulmonarius.*

Advantageously, the fermented powder comprises 0.005% to 0.30% by weight fungal β-glucans, preferably 0.010% to 0.18% by weight fungal β-glucans, more preferably 0.030% to 0.13% by weight fungal β-glucans, based on the total weight of the fermented powder. Fungal β-glucans in the fermented powder are preferably quantified as described above, in particular by following the method set forth in Example 1.2.

Advantageously, the fermented powder comprises 0.01 U to 50 U laccase by gram of the fermented powder, preferably 0.05 U to 40 U laccase by gram of the fermented powder, more preferably 0.10 U to 35 U laccase by gram of the fermented powder. Laccase activity of the fermented powder is preferably determined as described above, in particular by following the method set forth in Example 2.3.

Advantageously, the fermented powder comprises 3% to 40% by weight proteins, preferably, 5% to 35% by weight proteins, more preferably, 7% to 30% by weight proteins, based on the total weight of the fermented powder. Protein content can be determined by the Kjeldahl or Dumas method with a nitrogen factor of 6.25.

Advantageously, the fermented powder comprises 0.10% to 20% by weight fat, preferably, 0.50% to 15% by weight fat, more preferably, 1.0% to 10% by weight fat, based on the total weight of the fermented powder. Fat content can be determined by the Soxhlet extraction method.

Advantageously, the fermented powder comprises 20% to 80% by weight dietary fiber, preferably 25% to 75% by weight dietary fiber, more preferably 30% to 70% by weight dietary fiber, based on the total weight of the fermented powder. Dietary fiber content can be determined by the Prosky method, for example by following the Total Dietary Fibers AOAC 985.29 method.

Advantageously, the fermented powder comprises 1% to 60% by weight metabolizable carbohydrates, preferably 2% to 50% by weight metabolizable carbohydrates, more preferably 5% to 45% by weight metabolizable carbohydrates, based on the total weight of the fermented powder.

By "metabolizable carbohydrates", it is intended the fraction of carbohydrates that can be digested by human enzymes. On the contrary, dietary fiber represents the fraction of carbohydrates that cannot be digested by human enzymes. Therefore, the metabolizable carbohydrate content is calculated as the total carbohydrate content minus the dietary fiber content. Total carbohydrate content is preferably determined by difference, i.e. by subtracting protein, lipid, ash, moisture and dietary fiber contents from 100. The ash content can be determined according to NF V 18-101 (combustion) and the moisture content can be determined by steaming at 70°C under reduced pressure.

Advantageously, the fermented powder is obtained similarly to the process described in WO2018101844. In particular, the fermented powder can be obtained by the following process:
a) providing a plant material having a moisture level between 45% and 95%, optionally mixed with a food grade nitrogen source such as ammonium sulphate, glutamic acid, yeast extract, peptone and/or di-ammonium phosphate,
b) sterilising the plant material of a), such as preferably, at about 100 kPa and about 121°C for about 20 min,
c) inoculating the sterile plant material with edible higher fungi, such as preferably with a white rot and/or brown rot fungi at an amount of 0.1-10% w/w of the plant material,
d) incubating the inoculated plant material for a period of time to produce a fermented plant material, preferably at 18°C-40°C for a period of 5-50 days, more preferably, at 25°C-30°C for 14 to 35 days,
e) drying the fermented plant material of d), preferably at 30°C-70°C; and
f) milling the dried, fermented plant material to form a fermented powder.

The plant material and the fungi are as described above. In particular, the plant material is preferably grape or apple, and the fungi are preferably *Pleurotus pulmonarius.*

Advantageously, the fermented powder is stored under appropriate conditions until use.

Advantageously, the fermented powder is stored at room temperature (about 21°C) under vacuum, or at about 4°C or at about -20°C, optionally under vacuum, preferably at about 4°C or at about -20°C, more preferably at about -20°C.

Advantageously, when the fermented powder is stored "under vacuum", it is stored at a pressure equal to or less than 1000 Pa, preferably equal to or less than 100 Pa, more preferably equal to or less than 30 Pa, even more preferably equal to or less than 5 Pa.

In the process for clarifying a liquid according to the invention, the step of incubating the liquid with laccase and fungal β-glucans can advantageously be carried out at a temperature comprised between 20°C and 70°C.

Preferably, the incubation step is carried out at a temperature between 20°C and 60°C, more preferably, between 25°C and 55°C, even more preferably, between 30°C and 50°C, for example at 40°C.

If the incubation step is carried out at a temperature between 20°C and 70°C, then, the process according to the invention may include, after the incubation step, a step of cooling the liquid. Preferably, by cooling at a temperature of less than 10°C, more preferably less than 6°C, even more preferably between 0°C and 2°C, for a sufficient period of time (such as at least 5 min). For example, the cooling step can be carried by cooling on ice during 5 min.

Advantageously, the incubation step is carried out by stirring, preferably, between 2 and 100 rpm (revolutions per minute), more preferably, between 5 and 50 rpm, even more preferably, between 10 and 30 rpm, such as about 20 rpm.

Preferably, the incubation step is carried out during 30min to 20h, more preferably, 45 min to 13h, even more preferably, 1h to 6h.

Advantageously, the clarification step is carried out at a pH comprised between 1 and 7, preferably between 2 and 6, more preferably between 3 and 5, even more preferably of about 4.

Alternatively, the process for clarifying a liquid according to the invention, can further comprise an enzyme inactivation step after the step of incubating the liquid.

The enzyme inactivation step stops the incubation step and then the clarification of the liquid, by inactivating the laccase in the beverage after its clarification. The skilled person knows the conditions to achieve enzyme inactivation.

Advantageously, the enzyme inactivation is carried out by heating, by pasteurizing or by adding clay minerals as bentonite into the liquid or into the liquid after the clarification step. Preferably, clay minerals are removed after the enzyme inactivation by filtration, centrifugation or sedimentation.

Preferably, when the enzyme inactivation is carried out by heating, heating is preferably performed at a temperature of at least 80°C, more preferably at least 85°C, even more preferably at least 90°C, such as at 95°C, for a sufficient period of time to achieve enzyme inactivation (such as at least 5 min). For example, the enzyme inactivation can be carried out by heating for 5 min at 95°C.

Alternatively, the enzyme inactivation can be carried out by pasteurization. The skilled person knows the conditions to pasteurize; for example, pasteurization can be performed by heating 15 s at 95°C.

Advantageously, the enzyme inactivation (preferably, heating or pasteurization step) is immediately followed by a cooling step.

Preferably, by cooling at a temperature of less than 10°C, more preferably less than 6°C, even more preferably between 0°C and 2°C, for a sufficient period of time (such as at least 5 min). For example, the cooling step can be carried by cooling on ice during 5 min.

Advantageously, the process for clarifying a liquid according to the invention, can further comprise a solid-liquid separation step after the step of incubating the liquid.

The solid-liquid separation step is preferably carried out after the enzyme inactivation step or, when a cooling step is implemented after this cooling step.

Preferably, the solid-liquid separation step is performed by decanting, centrifuging and/or filtrating, more preferably, by centrifuging and/or filtrating, even more preferably, by centrifuging.

Advantageously, centrifuging is performed at least at 1,000 rpm, preferably, at least at 5,000 rpm, more preferably, at least at 10,000 rpm, even more preferably, between 10,000 rpm and 15,000 rpm, for a sufficient period of time to achieve a solid liquid separation (such as at least 5 min).

In a specific embodiment, the process for clarifying a liquid consists of the following steps:
- providing a plant material,
- contacting laccase and fungal β-glucans with the plant material,
- obtaining the liquid from said plant material,
- preparing a beverage from the liquid obtained from said plant material,
- optionally, contacting laccase and fungal β-glucans with the beverage,
- incubating the beverage comprising laccase and fungal β-glucans to clarify the liquid,
- inactivating the enzyme,
- optionally, cooling the beverage,
- filtrating the beverage, in order to retain the remaining solids.

The invention also relates to the use of laccase and fungal β-glucans for clarifying a liquid, said liquid being obtained from a plant material.

The laccase, fungal β-glucans, liquid and plant material are such as described above, including the advantageous features and preferred embodiments.

In particular, the clarified liquid advantageously has a relative total phenolic content of less than 98%, preferably, less than 95%, more preferably, less than 90%, with respect to the liquid to be clarified which has a 100% total phenolic content.

The invention also relates to a liquid or beverage selected from the group consisting of juices and plant-based milks comprising fungal β-glucans.

Said liquid or beverage is obtained according to the process of clarification according to the invention.

Advantageously, said liquid or beverage is obtained according to the process of clarification according to the invention, wherein the clarification is performed using a fungal fermented powder. Said fungal fermented powder may be used at 5,5 wt%. Preferably, Rosaceae fermented powder, Cereal fermented powder, Berry fermented powder, Apiaceae fermented powder, Actinidiaceae fermented powder, Amaranthaceae fermented powder, Amaryllidaceae fermented powder, Asteraceae fermented powder, Brassicaceae fermented powder, Cucurbitaceae fermented powder, Fabaceae fermented powder, Poaceae fermented powder, Solanaceae fermented powder, Rutaceae fermented powder, Chenopodiaceae fermented powder, Asparagaceae fermented powder, Concolculaceae fermented powder, Ericaceae fermented powder, Grossulariaceae fermented powder, Malvaceae fermented powder and Polygonaceae fermented powder. More preferably, said fungal fermented powder is selected from the group comprising Rosaceae fermented powder and Cereal fermented powder.

Thus, the invention also relates to a liquid or beverage comprising a fermented powder.

In particular, the beverage is a clarified beverage which advantageously has a relative total phenolic content of less than 98%, preferably, less than 95%, more preferably, less than 90%, with respect to the beverage to be clarified which has a 100% total phenolic content.

It should be noted that, when laccase is deactivated, laccase activity cannot be detected in the clarified liquid or beverage.

Preferably, the minimal amount of soluble fungal β-glucans in the clarified beverage is 10mg/L, preferably 20mg/L, preferably 30 mg/L, preferably 40mg/L, preferably 50mg/L, preferably 60mg/L, preferably 70mg/L, preferably 80mg/L, preferably 90 mg/L, preferably 100 mg/L, more preferably 150 mg/L, even more preferably 200 mg/L.

More preferably, the minimal amount of soluble fungal β-glucans contained in a plant-based milk is 90mg/L, and the minimal amount of soluble fungal β-glucans contained in a juice is 100mg/L.

Advantageously, the maximal amount of soluble fungal β-glucans in the clarified beverage is 50 g/L.

The amount of soluble fungal β-glucans can be measured by different methods. In a non-limiting way, the method can be, for example, a measure of the absorbance of the liquid, an enzyme immunoassay, calcofluor fluorescence assay. In one embodiment, the amount of soluble fungal β-glucans in the liquid is evaluated by the measure of the absorbance of the liquid according to a protocol adapted from Ko and Lin, 2004 with aniline blue as dye.

The adaptation is for a liquid instead of a powder: instead of weighting the powder and extracting 2 hours at 65°C in NaOH 1M, the liquid is diluted using NaOH to the same final concentration of 1M. Dilution is optimized to be in the range of the standards:
- For the extraction of β-glucans from clarified juices, dilution 4 times in 1 M NaOH,
- For the extraction of β-glucans from plant-based milks, dilution 10 times in 1M NaOH.

After this extraction step, the tubes are centrifuged before quantification of β-glucans to avoid interferences with absorbance measurement.

The liquid to be clarified, the beverage, the fermented powder and the fungal β-glucans are as described above, including the advantageous features and the preferred embodiments.

The invention will be better understood in the light of the following examples, given by way of illustration, with reference to:
- Figure 1 that is a diagram illustrating the total phenolic content (TPC) of juice after clarification treatment at 30°C with laccase and/or fungal β-glucans relatively to the blank for which the value was set at 100%;
- Figure 2 that is a diagram illustrating the turbidity, measured by absorbance at optical density of 650nm (OD650nm) after clarification treatment at 30°C;
- Figure 3 that is a diagram illustrating the total phenolic content (TPC) of juice after clarification treatment at 50°C with laccase and/or fungal β-glucans relatively to the blank for which the value was set at 100%.

### Example 1: Synergy with laccase and β-glucans for clarifying a juice

### 1.1 Apple juice extraction

Fresh apples were bought in a supermarket (Fuji variety). These fresh apples were stored in the fridge at 4°C prior to use.

To produce unclarified raw juice, apples were washed with tap water, cut in pieces and the seeds were removed. The raw juice was produced using the juice extractor Aicok KS-1503.

After pressing, the raw juice was characterized by its pH, brix and acidity (measured by Atago Pocket Brix-Acidity meter Multi Fruits) to ensure a good consistency. A calibration of the pH meter was carried out in a standard way using three solutions with a known pH of 4, 7 and 10. No enzymatic treatment was used at this stage. The apple raw juice implemented in this Example 1 has a pH of 3.8, 13.2 °Bx (degrees Brix) and an acidity of 5.8% (acidity relates to the concentration of acid compounds in the juice, and is expressed in grams per 100 mL, *i.e.* a juice having an acidity of 5.8% means that there are 5.8 grams of acid compounds per 100 mL of juice).

The juice is kept maximum 5 days at 4°C prior to its clarification treatment.

### 1.2 Laccase activity and β-glucan concentration

A solution of laccase from *Trametes versicolor* (Sigma Aldrich) was prepared at 10 g/L in 200 mM sodium acetate buffer pH 5. The laccase activity was measured by monitoring the 2,2'-azinobis(3-ethylbenzthiazoline-6-sulfonate) (ABTS) oxidation at 420 nm (extinction coefficient 36 L.mmol⁻¹.cm⁻¹) as described by Johannes and Majcherczyk, J. Biotechnol. 78 (2000) 193-199. Activity was assayed with 0.4 mM ABTS in 200 mM sodium acetate buffer pH 5 and room temperature (about 21°C) before each experiment.

The fungal β-glucan from *Piptoporus betulinus* (Carbosynth, product YG165864) was used for the experiment. The amount of soluble fungal β-glucan was quantified using aniline blue method and the protocol from Ko and Lin, J. Agric. Food Chem., 2004, 52(11):3313-8. The soluble fungal β-glucan were extracted from 300 mg of powder (Carbosynth, product YG165864) using 40 mL of 1M NaOH. The solution was incubated 2 hours in a water-bath at 65°C. To recover the supernatant containing the extracted fungal β-glucan, a centrifugation step was performed 10 min at 4.149 g-force and 25°C. 300 µL of this supernatant was analyzed: it was first incubated 30 min at 50°C, then 630 µL of the aniline blue dye mix (40 volumes of 0.1% aniline blue (Sigma Aldrich) + 21 volumes of 1M HCI + 59 volumes of 1M Glycine/NaOH buffer pH 9.5) was added and the mix was incubated 30 min at 50°C to form the 1,3-β-glucan-fluorochrome complex. Prior to fluorescence measurement, the tubes were incubated 30 min at room temperature (about 21°C) and then fluorescence intensity was measured (emission wavelength 502 nm, excitation wavelength 398 nm). The fungal β-glucan concentration was calculated based on a curve made with curdlan standards (from 0 to 17.5 µg/mL). It was determined that this extract of β-glucan from *Piptoporus betulinus* contains at least 20% by weight of fungal β-glucan, with respect to the total weight of the extract.

### 1.3 Analyses of the raw juice to be clarified

The supernatant is subjected to the quantification of total phenolic compounds as described in the protocol of Magalhaes et al., Talanta 83 (2010) 441-447. In brief, 50 µL of Folin-Ciocalteu reagent diluted 5 times is put in polystyrene microplate. 50 µL of raw juice supernatant diluted 20 times is added, then 100 µL of 0.35M NaOH is added. Microplate is incubated 3 min at room temperature (about 21°C) and absorbance is read at 760 nm using the plate reader Infinite 200 PRO (Tecan). The standard used is gallic acid.

### 1.4 Clarification treatment

The apple raw juice is equilibrated at the temperature of the experiment for around 5-10 min. The clarification agent is added to the juice; or the juice is added to the clarification agent already weighted in tubes. Laccase was put at a final concentration of 0.06 U/mL for clarification treatment. For the clarification experiment, a solution of β-glucan from *Piptoporus betulinus* was prepared at 10 g/L in distilled water. To solubilize the glucans and remove unsoluble compounds, the solution was heated 5 min at 95°C, vortexed, and centrifuged. The supernatant is the β-glucan solution. Based on the previous measure (see 1.2 above), it is considered that the β-glucan concentration in the β-glucan powder from *Piptoporus betulinus* is 20% by weight; therefore, the β-glucan solution comprises 2 g/L of β-glucan. This β-glucan solution was added to the apple juice so that the β-glucan concentration in the juice was 0.002 g/L (which corresponds to 0.01 g of β-glucan powder from *Piptoporus betulinus* per liter of juice).

A blank without clarification agent is already included in a set of experiment to be used as reference.

The reference conditions for the clarification treatment are: 40°C; 20 RPM stirring; 1 hour. Typically, 5 mL of juice is treated in Falcon 15 mL tubes. Other temperatures were tested; from 30 to 50°C. The tubes are incubated at the temperature of the experiment and stirred over 1 hour. Then, the clarification treatment is stopped by enzyme inactivation using a heating step of 5 min at 95°C (industrially this heating step is replaced by pasteurization, generally 15 seconds at 95°C). The tubes are cooled on ice during 5 min then centrifuged at 13,200 RPM (revolutions per minute) during 5 min, and the supernatant is analyzed. Alternatively, the centrifugation step could be replaced by filtration.

### 1.5 Results

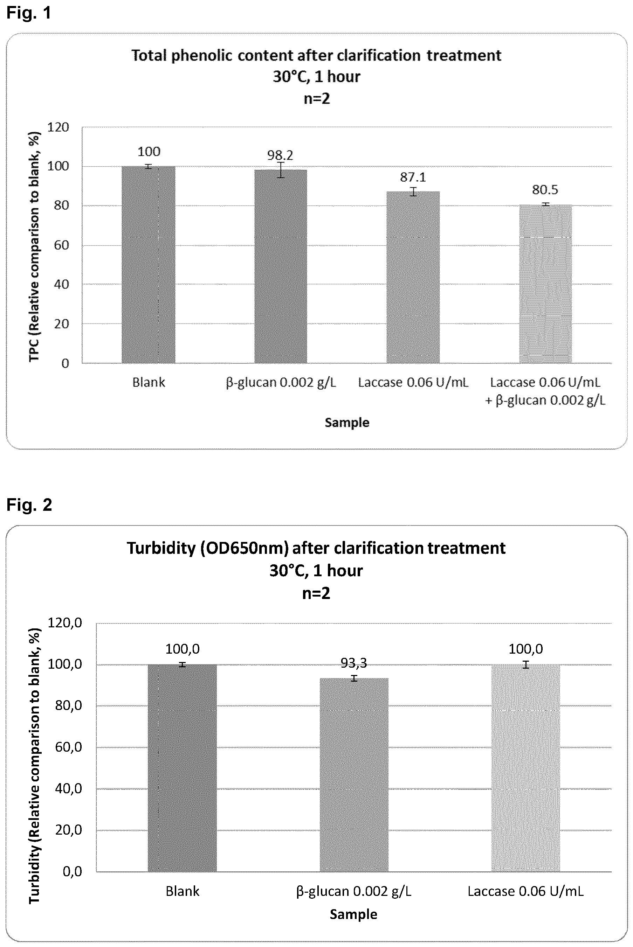

The results on the total phenolic content are provided in Table 1 and Fig. 1.

β-glucans alone showed a poor effect on clarification, considering both turbidity and phenolic content. However, it has been noted that β-glucans added to the liquid to be clarified do not add to pre-existing turbidity (Fig. 2).

Laccase alone showed a better effect on the phenolic content, in comparison with β-glucans alone.

However, after 1h at 30°C, a synergistic effect on total phenolic content was observed with laccase and β-glucan:

**Table 1: Total phenolic content of apple juice after clarification at 30°C with laccase and/or β-glucans**

| **Sample** | **Total phenolic content (mg/L equivalent gallic acid) Mean (n=2)** | **Relative percentage of total phenolic content with respect to blank** |
|---|---|---|
| Blank | 134.9 | - |
| β-glucan 0.002 g/L | 132.5 | -1.8% |
| Laccase 0.06 U/mL | 117.5 | -12.9% |
| Laccase 0.06 U/mL + β-glucan 0.002 g/L (experimental result) | 108.7 | -19.4% |
| Laccase 0.06 U/mL + β-glucan 0.002 g/L (artificial sum of the results) | 115.0 | -14.7% |

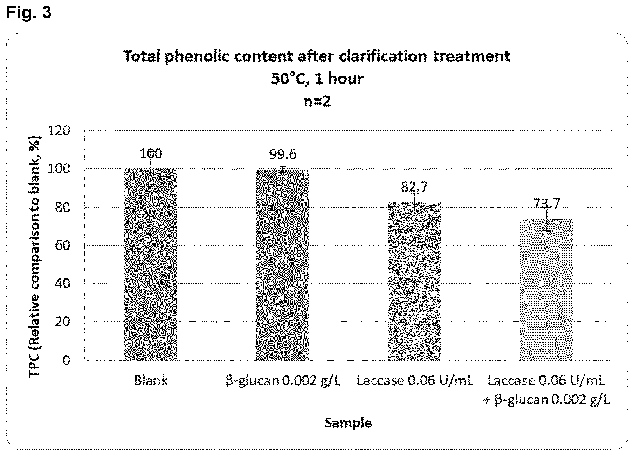

The same trend was observed after 1h at 50°C with the 0.002 g/L β-glucan concentration as shown in Table 2 and Fig. 3.

**Table 2: Total phenolic content of apple juice after clarification at 50°C with laccase and/or β-glucans**

| **Sample** | **Total phenolic content (mg/L equivalent gallic acid) Mean (n=2)** | **Relative percentage of total phenolic content with respect to blank** |
|---|---|---|
| Blank | 127.2 | - |
| β-glucan 0.002 g/L | 126.7 | -0.4% |
| Laccase 0.06 U/mL | 105.2 | -17.3% |
| Laccase 0.06 U/mL + β-glucan 0.002 g/L (experimental result) | 93.7 | -26.3% |
| Laccase 0.06 U/mL + β-glucan 0.002 g/L (artificial sum of the results) | 104.7 | -17.7% |

For this clarification experiment, the ratio used between β-glucan concentration (0.002 g/L) and laccase activity (0.06 U/mL) in the juice is 0.03.

### Example 2: Clarification with a fermented powder

### 2.1 Apple juice extraction

The apple juice was prepared according to Example 1.1.

The apple juice implemented in this Example 2 has a pH of 4.0, 12.5 °Bx and an acidity of 9.8%.

### 2.2 Preparation of berry fermented powder

A berry fermented powder was prepared from grape pomace similarly to the process set forth in application US 2018/0146688 A1.

In particular, a mixture of wet grape pomace (45 to 95 wt% moisture) and nitrogen source (such as 1 wt% yeast extract) was heat-sterilized (100 kPa and 121°C for 20 min), then 0.5-5 wt% of *Pleurotus pulmonarius* was added (weight of fungus with respect to the total weight of the wet pomace). After an optional mixing step, incubation was carried out for 14 to 35 days at a temperature of 25 to 30°C. The fermented pomace was then dried (30-70°C) and milled to produce a berry fermented powder.

The berry fermented powder can be stored at room temperature (21°C) under vacuum until use, but is preferably stored at 4°C or -20°C until use.

### 2.3 Laccase activity and β-glucan concentration

First, fungal β-glucan content was measured using the same method with aniline blue than described previously (Example 1.2). It was determined that the berry fermented powder comprises 0.044% β-glucans by weight, with respect to the total weight of the berry fermented powder.

Then, laccase activity was measured after extraction of the enzymes: a solution of the fermented powder was prepared at 20 g/L in 200 mM sodium acetate buffer pH 5 and stirred 1 hour at 10 RPM 4°C. After centrifugation, the supernatant was assessed for laccase activity using the same assay than previously described with ABTS (Example 1.2). Laccase activity of the berry fermented powder is 0.15 U/g.

### 2.4 Clarification treatment and results

The apple juice was clarified following the method set forth in Example 1.4 (except that 1 mL of juice was treated in 2 mL Eppendorf tube instead of 5 mL in 15 mL Falcon tube), the berry fermented powder being added at 2% by weight with respect to the apple juice weight. This corresponds to a β-glucan concentration added to the juice of 0.009 g/L and a laccase concentration of 0.003 U/mL.

The clarification treatment was carried out during 1h and at either 30°C, 40°C, 45°C or 50°C.

The total phenolic content was determined as indicated in Example 1.3 and the results are shown in Table 3. Each condition of the following results was made in triplicate and is expressed relatively to the blank value (set to 100%).

**Table 3: Total phenolic content of apple juice after clarification with berry fermented powder**

| | **Total phenolic content** | |
|---|---|---|
| **Sample** | **Mean (mg/L eq Gallic acid) n=3** | **Standard deviation** |
| Blank | 100.0 | 1.3 |
| Berry fermented powder 2% - 30°C | 90.0 | 2.4 |
| Berry fermented powder 2% - 40°C | 85.0 | 2.7 |
| Berry fermented powder 2% - 45°C | 86.0 | 0.8 |
| Berry fermented powder 2% - 50°C | 84.6 | 1.0 |

The turbidity of the juice was also evaluated (Table 4): either 300 µL of raw juice supernatant (blank) or 300 µL of clarified juice supernatant are put in a polystyrene microplate and absorbance at 650 nm is measured. This value is correlated to the turbidity of the juice: the lower it will be, the higher the juice will be clear. Each condition of the following results was made in triplicate and is expressed relatively to the blank value (set to 100%).

**Table 4: Visual clarification (Absorbance at 650 nm) of apple juice after clarification with 2% of berry fermented powder at different temperatures**

| | **Absorbance at 650 nm** | |
|---|---|---|
| **Sample** | **Mean (n=3)** | **Standard deviation** |
| Blank | 100.0 | 3.8 |
| Berry fermented powder 2% - 30°C | 96.4 | 0.3 |
| Berry fermented powder 2% - 40°C | 71.6 | 1.3 |
| Berry fermented powder 2% - 45°C | 83.0 | 1.3 |
| Berry fermented powder 2% - 50°C | 84.9 | 3.8 |

The results show that it is possible to use a fermented powder comprising laccase and β-glucans to clarify a juice. Indeed, reductions of the Total Phenolic Content and of the turbidity are observed when the fermented powder is added to the juice. Furthermore, the maximum Total Phenolic Content decrease is observed starting from 40°C with only 1 hour treatment is applied.

### Example 3: Clarification of a grape juice, when laccase and β-glucans are contacted directly with the grape pulp

An alternative process for clarification is disclosed below.

First, the grape must is extracted from previously cleaned grapes by crushing those grapes.

Then, laccase and β-glucans are contacted with the grape pulp and the whole is mixed.

Afterwards, the grape juice is recovered by pressing the grape pulp. Thus obtained, the grape juice contains laccase and β-glucans solubilized.

In order to enable clarification, the juice is put in optimum conditions, e.g. 40°C under stirring during 1 hour.

Finally, the juice is filtered or centrifuged and pasteurized.

## Claims

1. Process for clarifying a liquid obtained from a plant material, comprising a step of incubating the liquid with laccase and fungal β-glucans.

2. Process for clarifying a liquid according to claim 1, comprising a step of contacting laccase and fungal β-glucans with the plant material, before the plant material is processed into a liquid.

3. Process for clarifying a liquid according to claim 1 or claim 2, comprising a step of contacting laccase and fungal β-glucans with the liquid obtained from the plant material.

4. Process for clarifying a liquid according to claim 3, wherein the liquid is a beverage.

5. Process for clarifying a liquid according to claim 4, wherein the beverage to be clarified is a juice, beer or wine.

6. Process for clarifying a liquid according to any one of claims 1 to 5, wherein the fungal β-glucan / laccase ratio is comprised between 0.001 g/kU and 5.0 g/kU.

7. Process for clarifying a liquid according to any one of claims 1 to 6, wherein laccase and fungal β-glucans are provided under the form of a fermented powder.

8. Process for clarifying a liquid according to any one of claims 1 to 7, wherein the step of incubating the liquid with laccase and fungal β-glucans is carried out at a temperature comprised between 20°C and 70°C.

9. Process for clarifying a liquid according to any one of claims 1 to 8, further comprising an enzyme inactivation step after the step of incubating the liquid.

10. Process for clarifying a liquid according to any one of claims 1 to 9, further comprising a solid-liquid separation step after the step of incubating the liquid.

11. Use of laccase and fungal β-glucans for clarifying a liquid, said liquid being obtained from a plant material.

12. Liquid selected from the group consisting of juices and plant-based-milks comprising fungal β-glucans.
